# EUROPEAN PATENT APPLICATION

(11) **EP 4 108 330 A1**
(43) Date of publication of application: **28.12.2022**
(21) Application number: 21181054.4
(22) Date of filing: 23.06.2021
(51) Int. Cl.: B01L 3/00, G01N 33/48

(54) **MICROFLUIDIC DEVICE**

(71) Applicant: Sensirion AG, 8712 Stäfa (CH)
(72) Inventor: Winger, Martin, 8712 Stäfa (CH); Streiff, Matthias, 8032 Zürich (CH); Huber, Deborah, 8048 Zürich (CH)

(57) **Abstract**

The present invention relates to a microfluidic device (1) that is configured to determine a concentration of a target analyte (2) in a host liquid (3) using functionalized beads (4), the device (1) comprising: at least a receptacle (6) that is configured to receive the host liquid (3) and the functionalized beads (4), the receptacle (6) being implemented to have a closed surface (7), in relation to which a functionalized surface (9) is provided that is in communication with the host liquid (3), and to define a boundary surface (11) in respect of the host liquid (3), and an ultrasonic transducer (16) that is accessible to the microfluidic device (1) when it is in use, which can be positioned relative to a region of the receptacle (6) such as to produce acoustic waves in the host liquid (3) when it is operated, wherein: at least an acoustic boundary condition configured at a distinct region of the receptacle (6) and an operational frequency at which the ultrasonic transducer (16) is operated depending on at least a dimensional configuration (14, 15) of the receptacle (6), collectively define respective first and second modes of device operation, in which first and second acoustic wave configurations are correspondingly produced, so that the functionalized beads (4) are positioned differently in the receptacle (6) in respective first and second incubation events.

## Description

### Technical Field

The present invention relates to a microfluidic device and corresponding method for determining a concentration of a target analyte in a host liquid using functionalized beads.

### Prior Art

US2015044097 A1 discloses a filtration device for assays that can be used for filtering blood and attaching analytes in the blood to magnetic particles. Using the magnetic properties of the magnetic particles, the analytes are bound to an analyzing surface where they are counted. The results can then be displayed.

US2018362918 A1 discloses a device for separating particulates from a host fluid using an acoustic standing wave.

US2002112541 A1 discloses an ultrasonic force differentiation assay in which an ultrasonic energy source is used to provide a variable force for measuring the binding forces between molecular entities and for sensing the presence of an analyte in a test sample.

### Summary of the invention

According to an embodiment of a first aspect of the present invention, there is provided a microfluidic device that is configured to determine a concentration of a target analyte in a host liquid using functionalized beads, the device comprising: at least a receptacle that is configured to receive the host liquid and the functionalized beads, the receptacle being implemented to have a closed surface, in relation to which a functionalized surface is provided that is in communication with the host liquid, and to define a boundary surface in respect of the host liquid, and an ultrasonic transducer that is accessible to the microfluidic device when it is in use, which can be positioned relative to a region of the receptacle such as to produce acoustic waves in the host liquid when it is operated, wherein: at least an acoustic boundary condition configured at a distinct region of the receptacle and an operational frequency at which the ultrasonic transducer is operated depending on at least a dimensional configuration of the receptacle, collectively define respective first and second modes of device operation, in which first and second acoustic wave configurations are correspondingly produced, so that the functionalized beads are positioned differently in the receptacle in respective first and second incubation events.

In an embodiment of the present invention, first and second incubation events may be controlled according to corresponding first and second modes of device operation. In this way, and rather than being dependent on assay/device orientation, direction of gravitational force and/or size/weight of specifically bound particles, the concentration of the target analyte in the host liquid may be determined with increased accuracy.

Via first and second acoustic wave configurations that are respectively produced in the first and second modes of device operation, a positioning of the functionalized beads within the receptacle is controllably changeable during respective first and second incubation events. In the first incubation event, the functionalized beads are levitated away from the functionalized surface and in the second incubation event they are propagated to and bind at the functionalized surface. Targeted binding events involving either one of the target analyte particles and the functionalized beads during the first and/or second incubation event are supported by an embodiment of the present invention. Accordingly, the concentration of the target analyte in the host liquid may be determined with increased accuracy.

Preferably, the acoustic boundary condition comprises one of: an acoustic impedance change across the boundary surface of the host liquid, an acoustic impedance change across the closed surface of the receptacle, and a geometry of the boundary surface.

This feature imparts the advantages of versatility and flexibility to an embodiment of the present invention since operation in the first and second modes of device operation is not restricted to changing one or a specific acoustic boundary condition.

Desirably, the ultrasonic transducer is operably coupled to a timer that is configured so that the ultrasonic transducer is operated for a predetermined time-period in the first mode of device operation, thereby to facilitate the first incubation event occurring for the same time duration.

Because of this feature, the ultrasonic transducer may be operated for a time-period that accounts for the specific adsorption kinetics and/or diffusivity of the target analyte particles, which characteristics may be relevant in the first or second incubation event. Thus, this feature may facilitate the concentration of the target analyte in the host liquid to be determined with increased accuracy and in a tailored way.

Preferably, the ultrasonic transducer is configured to be operated at a resonant frequency in the first mode of device operation.

During the first incubation event, acoustophoresis forces are used to keep the functionalized beads levitated above and relatively further away from the functionalized surface. Here, they either engage in specific binding with the target particles or they allow for the target analyte particles to diffuse to, and specifically bind at, the functionalized surface Accordingly, this feature may extend the advantages of the first incubation event being performed with increased control and efficiency and for the concentration of the target analyte to be determined with increased accuracy.

Desirably, a medium is provided to interface with the boundary surface in the first mode of device operation, to which arrangement is associated an acoustic impedance mismatch across the boundary surface.

This feature lends the advantage that the boundary surface assumes a reflective quality, whereby the extent to which it is reflective depends on the difference in acoustic impedance between the host liquid and the medium that is provided above it. This feature also provides the advantage of ease of implementation as a physical reflector/reflector unit for acoustic waves is not required in an embodiment of the present invention. Regarding this feature, air is preferably provided to interface with the boundary surface of the host liquid because the relatively sharp discontinuity in acoustic impedance associated to a transition from the host liquid to air causes the boundary surface to be highly reflective to any impinging acoustic wave. This may be implemented with relative ease in that the the receptacle is simply left unobstructed at its open end.

Preferably, the functionalized beads comprise beads whose outer surface is coated with a bead functionalizing material comprising one of: at least a type of affinity body that facilitates specific binding with particles of the target analyte in the host liquid, and a type of a target analyte analog.

This feature supports different formats in which the first and second incubation events may occur. Thus, it further reinforces the advantages of versatility and flexibility in an embodiment of the present invention.

Desirably, the functionalized surface comprises a surface functionalizing material to which at least the functionalized beads may bind in the second incubation event and that is selected according to a functionalization of the functionalized beads.

Because the surface functionalizing material may be selected to be complementary to a choice of the bead functionalizing material, the second incubation event may be performed according to a chosen format and, therefore, with increased control and efficiency.

Preferably, at least a wall of the receptacle comprises at least a slot.

This feature facilitates increased lateral confinement and/or reduced loss of either of the first and second acoustic wave configurations, which are produced in relation to corresponding first and second incubation events.

Desirably, the receptacle comprises at least a conduit.

This feature may facilitate access to the receptacle for a specific purpose, which may be, to flush further liquid into the receptacle, for example. In addition, or alternatively, it may serve to link the receptacle outwardly to least another microfluidic device. This feature has the associated advantage that it is multi-functional and dedicated components need not be structurally incorporated into an embodiment of the present invention for the purposes that it may serve.

Preferably, an embodiment according to the first aspect of the present invention is configured to be incorporated in an array when it is in use.

This feature facilitates ease of integration and ease of determining the target analyte concentration in a mass testing environment.

Desirably, the ultrasonic transducer comprises an illumination access feature that is at least one of: a hole formed in the ultrasonic transducer that is at least partially aligned with an illumination source, and the ultrasonic transducer comprises a material that is transparent to radiation emitted by the illumination source.

To facilitate ease of counting/detecting the functionalized beads bound at the functionalized surface in the second incubation event, and performing this task with increased accuracy, the ultrasonic transducer may be provided in a configuration in which it comprises an illumination access feature. This feature may be integrated and/or fabricated with ease in respect of the ultrasonic transducer because it may take the form of a hole in the ultrasonic transducer and/or that the ultrasonic transducer comprises a material that is transparent to radiation emitted by the illumination source. Also, this feature enhances a capability of determining the target analyte concentration with increased accuracy, even if the ultrasonic transducer is portably positioned relative to different regions of the receptacle.

Preferably, the ultrasonic transducer is configured to be operated at an off-resonant frequency in the second mode of device operation.

This feature may extend the advantage that the second acoustic wave configuration that is correspondingly produced propagates the functionalized beads in a controllable and dedicated manner towards the functionalized surface where they engage in the second incubation event. This may contribute to determining the concentration of the target analyte in the host liquid with increased accuracy and efficiency.

Desirably, a tunable liquid lens is provided in relation to the boundary surface to facilitate changing the geometry thereof in the second mode of device operation.

This feature may be implemented with ease at the boundary surface. It may correspondingly extend the advantages of providing an alternative way in which switching to the second mode of device operation may be performed with ease in an embodiment of the present invention. Preferably, an impedance-matched material is provided in contact with one of the boundary surface and the closed surface of the receptacle, in the second mode of device operation, thereby to relatively match the acoustic impedance across that surface.

This feature offers the advantage that switching to the second mode of device operation is not restricted in respect of a fixed region of the receptacle. In fact, depending on a context of provision of an embodiment of the present invention, the second mode of device operation may be effectuated by interfacing one of the boundary surface and the closed surface with an impedance-matched material.

Desirably, the receptacle is configured to facilitate an increased volume of the host liquid in the receptacle, in the second mode of device operation, so that the boundary surface defined in relation to the first incubation event is immersed in the host liquid and the acoustic impedance across it is matched.

This feature facilitates yet another alternative to switching to the second mode of device operation and may extend associated advantages, including relative ease of implementation and adaptability to different contexts in which an embodiment of the present invention may be provided, for example, in an array format.

A corresponding method aspect is also provided, and so according to an embodiment of a second aspect of the present invention, there is provided a method of determining a concentration of a target analyte in a host liquid using functionalized beads, the method comprising the steps of: providing at least a receptacle that is configured to receive the host liquid and the functionalized beads, implementing the receptacle to have a closed surface, in relation to which a functionalized surface is provided that is in communication with the host liquid, and to define a boundary surface in respect of the host liquid, and positioning an ultrasonic transducer relative to a region of the receptacle and operating it to produce acoustic waves in the host liquid, the method further comprising the steps of: configuring at least an acoustic boundary condition at a distinct region of the receptacle and operating the ultrasonic transducer at an operational frequency depending on at least a dimensional configuration of the receptacle, which collectively facilitate operating in one of a first and second mode of operation, and correspondingly producing respective first and second acoustic wave configurations, so that the functionalized beads are positioned differently in the receptacle in respective first and second incubation events.

### Brief description of the drawings

Reference will now be made to the accompanying drawings in which:
Figure 1 schematically illustrates an embodiment of the present invention.
Figure 2 schematically illustrates a sandwich assay format in an embodiment of the present invention.
Figures 3A and 3B schematically illustrate a respective first and second competitive assay format in an embodiment of the present invention.
Figure 4 schematically illustrates a first mode of device operation according to an operational frequency of an ultrasonic transducer in an embodiment of the present invention.
Figure 5 schematically illustrates switching to a second mode of device operation by changing the operational frequency of the ultrasonic transducer.
Figure 6 schematically illustrates switching to the second mode of device operation by reducing an acoustic impedance mismatch across a boundary surface.
Figure 7 schematically illustrates switching to the second mode of device operation by matching the acoustic impedance across the boundary surface.
Figures 8A and 8B schematically illustrate a respective first and second mode of device operation by an acoustic impedance change across a closed surface.
Figure 9 is a top view of a different structural configuration of the ultrasonic transducer.
Figures 10A and 10B schematically illustrate a respective first and second mode of device operation by changing a geometry of the boundary surface.
Figure 11 schematically illustrates an alternative structural composition of a receptacle in an embodiment of the present invention.
Figure 12 shows an alternative geometry of the receptacle.
Figures 13 and 14 respectively show different structural configurations for the receptacle.

### Description of preferred embodiments

Within the description, the same reference numerals or signs have been used to denote the same parts or the like.

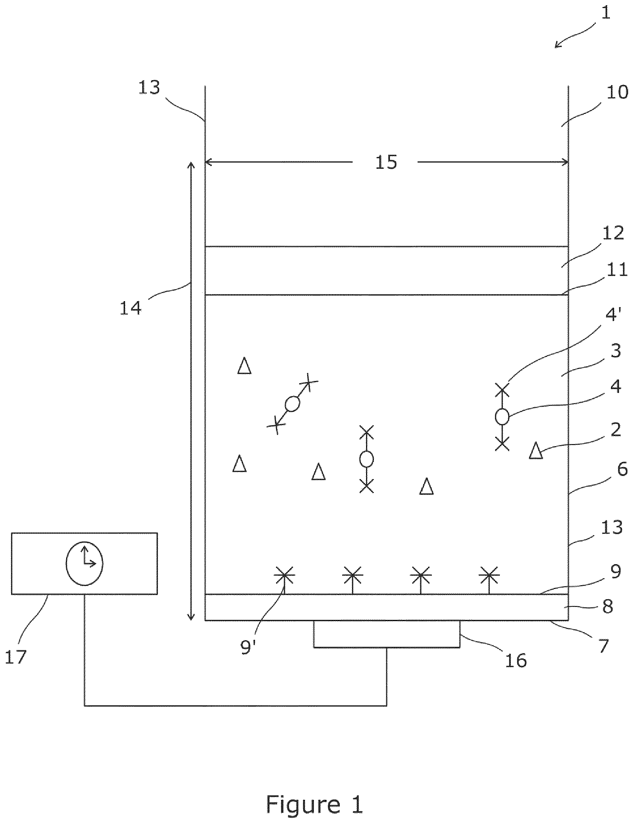

Reference is now made to Figure 1 that schematically illustrates a microfluidic device 1 according to an embodiment of the present invention, which may be configured to determine a concentration of a target analyte 2 in a host liquid 3.

The microfluidic device 1 comprises a receptacle 6 that is configured to receive the host liquid 3. It is implemented to have at least a closed surface 7 comprising a functionalized surface 9 that is in communication with the host liquid 3. The functionalized surface 9 may be implemented directly on top of the closed surface 7. Alternatively, it may be implemented as a part of a customized layer 8, comprising a sensor chip/integrated circuit. The customized layer 8 may be provided on top of the closed surface 7 or in a version where it comprises the closed surface 7, in full or in part.

The receptacle 6 may also comprise an open end 10 as shown in Figure 1 but this is not limiting, and an embodiment of the present invention may be closed at both ends.

The receptacle 6 is configured to define a boundary surface 11 that is defined in respect of the host liquid 3 when the microfluidic device 1 is in use, and that is disposed vertically opposite to the closed surface 7. The boundary surface 11 comprises an interface of the host liquid 3 in respect of any medium(s) 12 adjoining/interfacing with it.

The host liquid 3 is confined within walls 13 of the receptacle 6 having a specific wall height 14. The microfluidic device 1 is implemented by a specific choice of receptacle diameter 15. The receptacle 6 may be made of glass, a polymer or plastic such as, for example, polyethylene terephthalate (PET), polypropylene (PP), polycarbonate (PC), cyclic olefin copolymer (COC) or such like.

In an embodiment of the present invention, the host liquid 3 may comprise a biological sample 3, for example, a blood sample, containing particles of a target analyte 2, which may be a type of protein, antibody, bacteria, virus, or a smaller organic molecule such as a toxin or nucleic acid like DNA and RNA.

An ultrasonic transducer 16 is accessible to the microfluidic device 1 when it is in use. In an embodiment of the present invention, it is operably coupled in relation to a desired region of the receptacle 6 such as to produce acoustic waves in the host liquid 3 when it is operated. The ultrasonic transducer 16 may be provided in different formats in an embodiment of the present invention. It may be provided in a portable format as a separate component that can be portably positioned relative to the receptacle 6. In addition, or alternatively, it may be provided in a fixed format as an integral part of the receptacle 6 and/or fixed to the receptacle 6. Where it is provided as an integral part of the receptacle 6, and by way of example, the ultrasonic transducer 16 may be integrated within the customized layer 8 as a piezoelectric micromachined ultrasonic transducer. Figure 1 shows an arrangement of the ultrasonic transducer 16 where it may be positioned portably and/or fixed under the closed surface 7 of the receptacle 6. A timer 17 is operably coupled to the ultrasonic transducer 16 to operate it for a specific and controllable duration.

In an embodiment of the present invention, first and second incubation events may be controlled according to corresponding first and second modes of device operation. In this way, and rather than being dependent on assay/device orientation, direction of gravitational force and/or size/weight of specifically bound particles, the concentration of the target analyte 2 in the host liquid 3 may be determined with increased accuracy.

Via first and second acoustic wave configurations that are respectively produced in the first and second modes of device operation, a positioning of the functionalized beads 4 within the receptacle 6 is controllably changeable during respective first and second incubation events. In the first incubation event, the functionalized beads 4 are positioned away from the functionalized surface 9 and are levitated relatively closer to the boundary surface 11. In the second incubation event, the functionalized beads 4 are traversed to, and positioned at, the functionalized surface 9. Such controllable positioning of the functionalized beads 4 supports targeted binding events involving either one of the target analyte particles 2 and the functionalized beads 4 during the first and/or second incubation event. Accordingly, the concentration of the target analyte 2 in the host liquid 3 may be determined with increased accuracy.

A bead functionalizing material 4' and a surface functionalizing material 9' are coated on a corresponding outer/upper surface of the functionalized beads 4 and the functionalized surface 9 as can be seen from Figure 1. They collectively determine a format for the binding events that occur in the first and second incubations. The surface functionalizing material 9' facilitates specific binding of/involving the functionalized beads 4 to the functionalized surface 9 in the second incubation event and it is selected according to the choice of bead functionalizing material 4'.

Generally, the second incubation event in an embodiment of the present invention is designed such that the number or proportion of the functionalized beads 4 specifically bound to the functionalized surface 9, either directly or indirectly, may be determined by counting. Before such counting is done, particles that have not specifically bound to the functionalized surface 9 are removed by "washing"/flushing them away for increased accuracy of determining the target analyte concentration. The functionalized beads specifically bound to the functionalized surface 9 may then be analyzed and counted by using infrared radiation. The concentration of the target analyte 2 is accordingly determined from the count.

As will be described here below, either of the bead functionalizing material 4' and the surface functionalizing material 9' may comprise at least a type of affinity body 5, which facilitates specific binding of the functionalized beads 4 and/or the functionalized surface 9 with particles of the target analyte 2 in the host liquid 3. Examples of such affinity bodies 5 include, but are not limited to, antibodies - both monoclonal and polyclonal, aptamers, designed ankyrin repeat proteins (DARPins), molecularly imprinted polymers, and oligomers.

The different formats in which first and second incubation events occur in an embodiment of the present invention, according to a choice of the bead functionalizing material 4' and the surface functionalizing material 9', are now described.

### Sandwich assay format

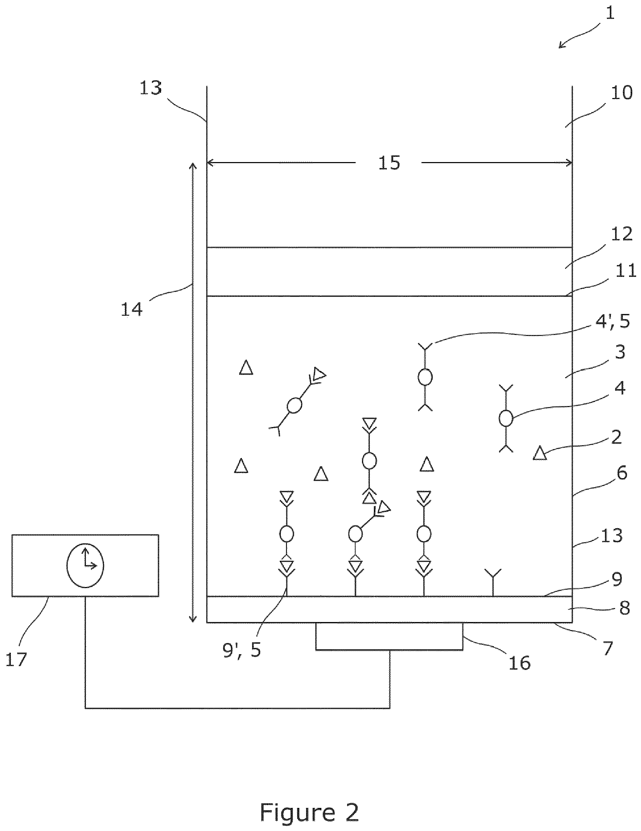

Referring now to Figure 2, which shows an embodiment of the present invention in which the bead functionalizing material 4' and the surface functionalizing material 9' comprise the same type of affinity body 5. This scenario is, of course, not limiting - the type of affinity body 5 may be selected to be different for each functionalization and broadly on account of facilitating specific binding with the target analyte particles 2.

In the first incubation event, the target analyte particles 2 in the host liquid 3 specifically bind to the affinity bodies 4',5 of the functionalized beads 4.

In the second incubation event, the specifically bound particles from the first incubation event incubate at the functionalized surface 9. In this respect, target analyte particles 2 in the complexes, resulting from the first incubation event, bind to the affinity bodies 9',5 of the functionalized surface 9. Thus, the functionalized beads 4 are indirectly bound to the functionalized surface 9 in a complex form.

This series of binding events comprises a sandwich-capture immuno-assay format in an embodiment of the present invention.

This format may present some complexity in that it involves two binding sites for the target analyte particles 2, this being in respect of both the first and second incubation events. However, it may have the associated advantages of increased sensitivity and/or accuracy with which the concentration of the target analyte 2 may be determined.

### 1^{st} competitive assay format

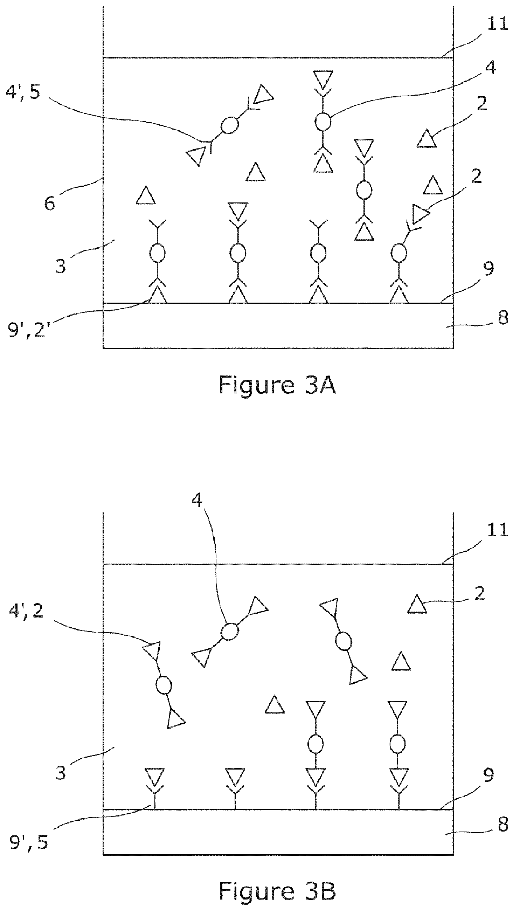

Referring now to Figure 3a, which shows an embodiment of the present invention in which: the bead functionalizing material 4' comprises a type of affinity body 5 and the surface functionalizing material comprises a target analyte analog 2'. In an embodiment of the present invention, a target analyte analog 2' comprises a particle that is deemed to be a copy of a target analyte particle 2 in that it has the same binding kinetics to the affinity body 5 as the target analyte particle 2. For ease of understanding, only the aspects relevant for/to the 1^{st} competitive assay format are shown in Figure 3 a.

In the first incubation event, the target analyte particles 2 in the host liquid 3 specifically bind to the affinity bodies 4', 5 of the functionalized beads 4.

In the second incubation event, the target analyte analogs 9', 2' specifically bind to the affinity bodies 4', 5 of the functionalized beads 4 that have not already bound to target analyte particles 2 in the first incubation.

This series of binding events comprises a 1^{st} competitive immuno-assay format in an embodiment of the present invention where the target analyte particles 2 and the target analyte analogs 2' respectively compete for the functionalized beads 4 in the first and second incubation events. In this case, the functionalized beads 4 are directly bound to the functionalized surface 9 via the affinity bodies 4', 5 used for their functionalization.

An advantage presented by this format is that, in contrast to the sandwich assay format, only one binding site is involved for the target analyte particles 2, this being in respect of the first incubation event. This format may present the advantages of increased speed, simplicity and/or flexibility with which the concentration of the target analyte 2 may be determined.

### 2nd competitive assay format

Referring now to Figure 3b, which shows an embodiment of the present invention in which: the bead functionalizing material 4' comprises target analyte particles 2 and the surface functionalizing material 9' comprises a type of affinity body 5. Again, only the aspects relevant for/to the 2^{nd} competitive assay format are shown in Figure 3b for ease of understanding.

In this format, the first incubation event is tailored to accommodate a diffusion and specific binding of the target analyte particles 2 in the host liquid 3 to affinity bodies 9', 5 at the functionalized surface 9. Because of the bead functionalizing material 4', 2 being identical to the target analyte particles 2, the functionalized beads 4 do not engage in any specific binding with the target analyte 2 in the host liquid 3 in the first incubation event.

The second incubation event is tailored for the functionalized beads 4 to specifically bind to sites on the functionalized surface 9 that are not already occupied by target analyte particles 2 from the first incubation event. Such targeted binding occurs between the target analyte particles 4', 2 used for functionalization of the functionalized beads 4 and available affinity bodies 9', 5 of the functionalized surface 9.

This series of binding events comprises a 2^{nd} competitive immuno-assay format in an embodiment of the present invention where the target analyte particles 2 in the host liquid 3 and the functionalizing target analyte particles 4', 2 on the functionalized beads 4 respectively compete for the functionalized surface 9 in the first and second incubation events.

In respect of the competitive assay formats, the use of 1^{st} and 2^{nd} do not denote any order of importance, advantage and/or hierarchy.

### First and second modes of device operation

In an embodiment of the present invention, first and second incubation events may be controlled according to corresponding first and second modes of device operation. Via first and second acoustic wave configurations that are respectively produced in the first and second modes of device operation, a positioning of the functionalized beads 4 within the receptacle 6 is changed: to be away from the functionalized surface 9 in the first incubation event and to be at the functionalized surface 9 in the second incubation event.

The first and second acoustic wave configurations are determined by a combination of an acoustic boundary condition configured at a distinct region of the receptacle 6, and an operational frequency at which the ultrasonic transducer 16 is operated in dependency on receptacle dimensions 14, 15. The acoustic boundary condition comprises an acoustic impedance change across at least one of: the boundary surface 11 of the host liquid 3 and the closed surface 9 of the receptacle 6. It may also comprise a geometry of the boundary surface 11.

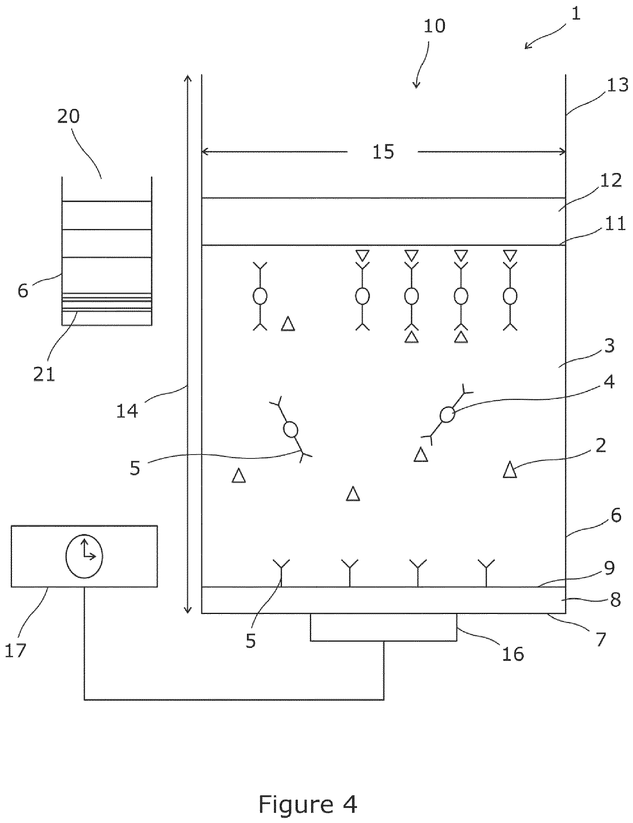

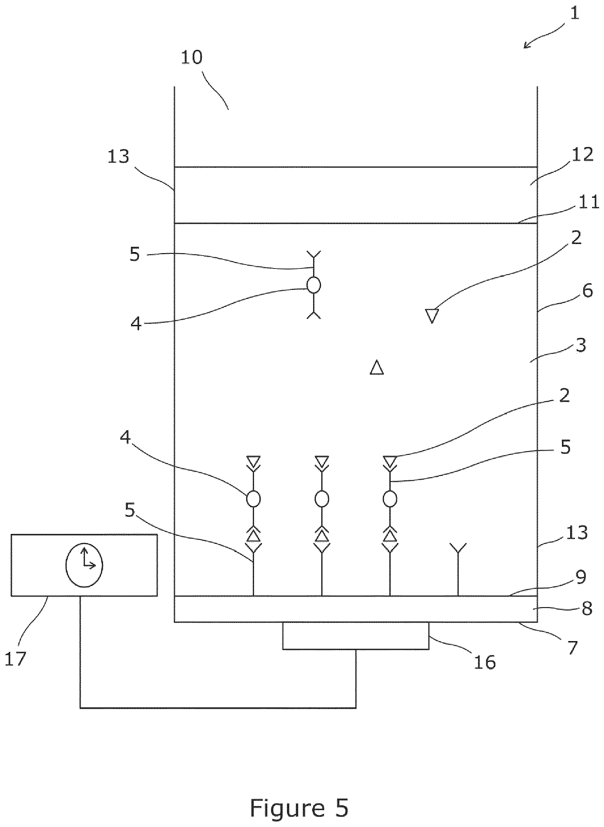

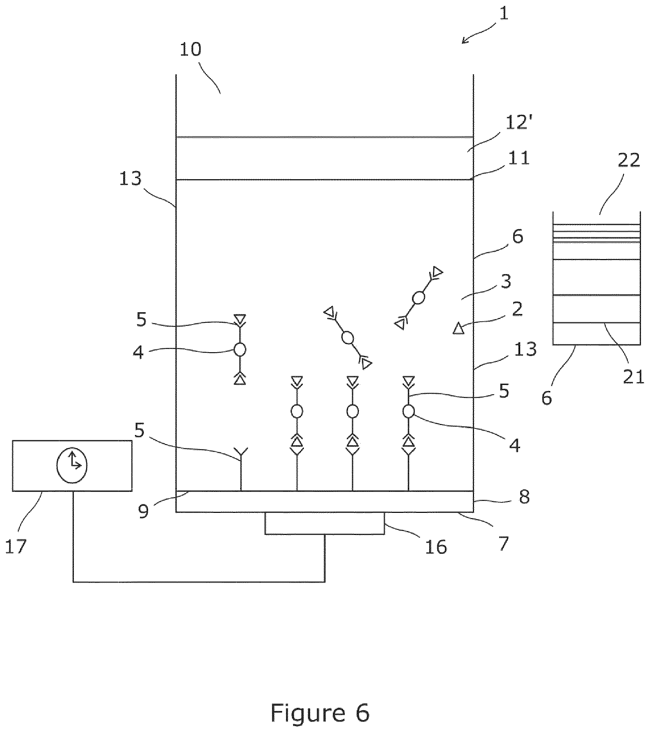

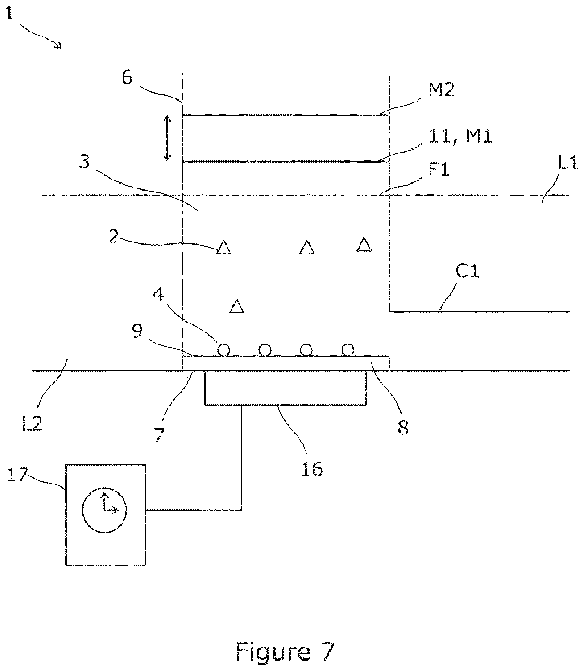

The first mode of device operation as shown in Figure 4 and the second mode of device operation as described with reference to Figures 5 and 6 will be described here below in respect of conducting the first and second incubation events according to the sandwich assay format of Figure 2. For ease of understanding, details on the functionalization of the functionalized beads 4 and/or the binding events that occur in different variations of the second mode of device operation as per any one of Figures 7, 8A, 8B, 10A and 10B are not shown in those drawings. Of course, the above-discussed sandwich assay format and different versions of the competitive assay formats may be imported and apply to these different variations as well.

### First mode of device operation

Referring to Figure 4, which schematically illustrates the first mode of device operation of the microfluidic device 1.

In this mode, an arrangement of providing a medium 12 to interface with the boundary surface 11 of the host liquid 3 is done on account of an associated acoustic impedance mismatch occurring across the boundary surface 11. Such a mismatch imparts a reflective quality to the boundary surface 11, whereby the greater the difference between the acoustic impedance of the medium 12 and that of the host liquid 3, the more reflective the boundary surface 11 will be for any impinging acoustic wave.

In an implementation of the first mode of device operation, a region above the host liquid 3 in the receptacle 6 is left unobstructed, i.e., the medium 12 comprises air. In this case, because the difference in acoustic impedance between the host liquid 3 and air 12 is relatively high, the boundary surface 11 is imparted the quality of increased reflectivity for any incident acoustic wave.

In the first mode of device operation, the ultrasonic transducer 16 is configured to be operated at a resonant frequency. Because of the boundary surface 11 being relatively highly reflective, a first acoustic standing wave configuration is produced. Its nodes, which are at potential energy minima, capture the functionalized beads 4 where they specifically bind with target analyte particles 2 as described with reference to Figure 2. The nodes are positioned relatively further away from the functionalized surface 9 and closer to the boundary surface 11 and so this is where the functionalized beads 4 are levitated in the first incubation event.

The inset 20 in Figure 4 pertains to the receptacle 6 and shows schematic isopotential lines/surfaces 21 of the first acoustic standing wave configuration depicting an acoustic potential energy landscape in the host fluid 3 in the receptacle 6. Where they are closer, there is a larger gradient in potential energy, that is, a larger acoustic force acts on the functionalized beads 4 there. In this first mode of operation, the potential energy minimum (zero force) is relatively further away from the functionalized surface 9 and closer to the boundary surface 11.

For an implementation of the microfluidic device 1 in which the receptacle wall height 14 and receptacle diameter 10 are each 2mm and the layer 8 comprises a silicon sensor chip having dimensions of 4mm by 4mm and a thickness of 350 microns, a high Q resonance mode is excited by operating the ultrasonic transducer 16 at 2MHz. Accordingly, a first acoustic standing wave configuration is produced that keeps the functionalized beads 4 levitated away from the functionalized surface 9 in the first mode of device operation. The dimensional configurations 14, 15, of the receptacle 6 are selected according to a microfluidics aspect and the operational frequency of the ultrasonic transducer 16 is chosen such that a wanted mode is excited.

In the first mode of device operation, the timer 17 is configured so that the ultrasonic transducer 16 is operated for a predetermined time-period, thereby to facilitate the first incubation event occurring for the same time duration.

For the sandwich assay and 1^{st} competitive assay formats respectively described with reference to Figures 2 and 3a, the ultrasonic transducer 16 may be operated for a time-period accounting for the specific adsorption kinetics of the target analyte particles 2 to the affinity bodies 9', 5 of the functionalized beads 4.

For the 2^{nd} competitive assay format described with reference to Figure 3b, the ultrasonic transducer 16 may be operated for a time-period accounting for: a diffusivity of the target analyte particles 2 towards the functionalized surface 9 and additionally for the specific adsorption kinetics of the target analyte 2 to the affinity bodies 9', 5 of the functionalized surface 9.

### Second mode of device operation

To switch from the first to the second incubation event, at least one of: an acoustic boundary condition and an operational frequency of the ultrasonic transducer 16 is changed. These variations have in common that they facilitate positioning of the functionalized beads 4 at the functionalized surface 9 in the second incubation event. They differ in the second acoustic wave configurations, which are correspondingly produced for propagating and/or holding the functionalized beads 4 at the functionalized surface 9.

### Changing the operational frequency of the ultrasonic transducer 16

Reference is now made to Figure 5 in which operation of the ultrasonic transducer 16 is switched from the resonant frequency described hereinabove with respect to Figure 4 to an off-resonant frequency. This is done by operating the ultrasonic transducer 16 at a frequency value that is relatively close to the resonant frequency. In relation to a bandwidth = resonant frequency/Q-factor, the off-resonant frequency is no more than three bandwidths, and more preferably, no more than one bandwidth away from the resonant frequency. The off-resonant excitation is such that a second, acoustic travelling wave configuration is generated, which propagates the functionalized beads 4 specifically bound to target analyte particles 2 in the first incubation event towards the functionalized surface 9.

### Changing an acoustic boundary condition

As discussed above, switching to the second mode of device operation may be done by changing an acoustic boundary condition. This may be implemented in relation to an acoustic impedance change across at least one of: the boundary surface 11 of the host liquid 3 and the closed surface 7 of the receptacle 6. It may also be implemented in relation to a geometry of the boundary surface 11.

### Acoustic impedance across the boundary surface

Altering the acoustic impedance across the boundary surface 11 facilitates switching to the second mode of device operation. Such an alteration may comprise either reducing the acoustic impedance mismatch or matching the acoustic impedance across the boundary surface 11.

### Reducing the acoustic impedance mismatch across the boundary surface

Referring to Figure 6, to switch to the second mode of device operation, an impedance-matched material 12' is provided in mechanical contact with the boundary surface 11 after a completion of the predetermined time-period associated with the first incubation.

The impedance-matched material 12' is chosen on account of having a relatively similar acoustic impedance as the host liquid 3. A reduced acoustic impedance mismatch occurs across the boundary surface 11 compared to the first mode of device operation, and an acoustic bridge is formed at the boundary surface 11. With this arrangement, the relatively high reflectivity of the boundary surface 11 is suppressed. Accordingly, a second acoustic standing wave configuration is produced with nodes positioned at the functionalized surface 9. This is illustrated by the inset 22 in Figure 6 - it pertains to the receptacle 6 and shows isopotential lines/surfaces 21 of the second acoustic standing wave configuration depicting an acoustic potential energy landscape in the host fluid 3 in the receptacle 6. The potential energy minimum (zero force) is located at the functionalized surface 9 and this is where the nodes of the second acoustic standing wave configuration are positioned. Complexes of the functionalized beads 4 and target analyte particles 2 from the first incubation event are captured by these nodes in the second incubation event.

The impedance-matched material 12' may comprise an elastomer, hydrogel and, where it is implemented with a liquid lens, it may comprise a host liquid 3 - oil film interface/meniscus tunable via an applied voltage.

### Matching the acoustic impedance mismatch across the boundary surface

Alternatively, switching to the second mode of device operation may be done by matching the acoustic impedance across the boundary surface 11. This is done by increasing a volume of the host liquid 3 in the receptacle 6 after a completion of the predetermined time-period associated with the first incubation. The volume of the host liquid 3 is increased by flushing liquid into the receptacle 6, which may comprise more host liquid 3, water or any other appropriate liquid.

Turning to Figure 7, during the first incubation event, the meniscus M1 of the host liquid 3 coincides with the boundary surface 11 as it is defined in the first incubation event. With the volume of the host liquid 3 being increased to switch to the second incubation event, a new meniscus M2 is defined, which is at a different height than the original meniscus M1. This can be clearly seen from Figure 7 where new meniscus M2 occurs higher up in the receptacle 6 as compared to meniscus M1. The originally defined boundary surface 11 is immersed in the host liquid 3 and the acoustic impedance across it is matched. This has the effect that the reflectivity of the originally defined boundary surface 11 is completely suppressed and that the resultant second acoustic standing wave configuration comprises nodes positioned at the functionalized surface 9. The functionalized beads 4 from the first incubation event are captured at these nodes in the second incubation event.

To flush liquid into the receptacle 6, and as can be seen from Figure 7, the receptacle 6 is provided with a conduit C1. Additional liquid may also be pushed into the receptacle 6 by increasing a pressure in the conduit C1, as exemplified by the moved menisci M1, M2. The receptacle 6 may comprise an inbuilt filter F1 where the functionalized beads 4 are retained when the flushing is performed.

In this example of an embodiment of the present invention, the microfluidic device 1 is provided in an array format where it is linked to other microfluidic devices 1/parts of the array via linking regions L1, L2.

The receptacle 6 in an embodiment of the present invention is not limited to the arrangement of the conduit C1 as shown in Figure 7. Indeed, any other arrangement and/or similar technical feature is encompassed within a scope of an embodiment of the present invention where a flushing of extra liquid into the receptacle 6 is done.

### Changing the acoustic impedance across the closed surface

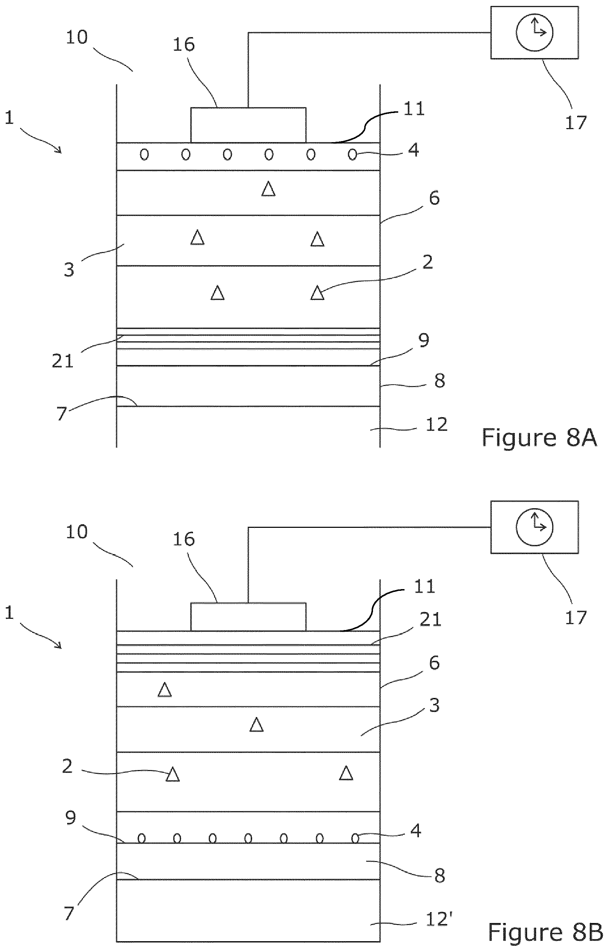

Switching to the second mode of device operation may be done via a combination of changing a positioning of the ultrasonic transducer 16 and the acoustic impedance across the closed surface 11. Figures 8A and 8B show respective first and second modes of device operation of this embodiment of the present invention. For ease of understanding, details on the functionalization of the functionalized beads 4, functionalized surface 9 and/or the binding events that occur in the first and second incubation are not shown. Of course, the above-discussed sandwich assay format and different versions of the competitive assay formats may be imported and apply to this implementation as well.

Figure 8A schematically illustrates the first mode of device operation. It differs from the first mode of device operation discussed with reference to Figure 4 in that the ultrasonic transducer 16 is positioned at the boundary surface 11 instead of at the closed surface 7 of the receptacle 6. The functionalized beads 9 are held closer to the boundary surface 11/away from the functionalized surface 9 due to a first acoustic standing wave configuration, which is produced by a combination of the ultrasonic transducer 16 being operated at a resonant frequency and a relatively high reflectivity of the closed surface 7. In this implementation, and as depicted by a spacing between the isopotential lines/surfaces 21 shown in Figure 8A, nodes occur closer to the boundary surface 11 for capturing and levitating the functionalized beads 4 there.

A large acoustic impedance mismatch occurs across the closed surface 7 due to a difference in acoustic impedance between the host liquid 3 and air 12 exterior to the receptacle 6 and in relation to the closed surface 7. Thus, a reflective quality is imparted to the closed surface 7 to any impinging acoustic wave.

After a predetermined time-period associated with the first incubation, a switch is performed to the second mode of device operation.

To switch to the second mode of device operation, and in contrast to the implementation of Figure 6, the impedance-matched material 12' is brought into contact with the closed surface 7 as shown in Figure 8B. It is chosen on account of having a relatively similar acoustic impedance to a material of fabrication of the closed surface 7. This has the effect that the reflectivity of the closed surface 7 is suppressed. Accordingly, a second acoustic standing wave configuration is produced with nodes positioned at the functionalized surface 9 where the functionalized beads 4 are captured in the second incubation event.

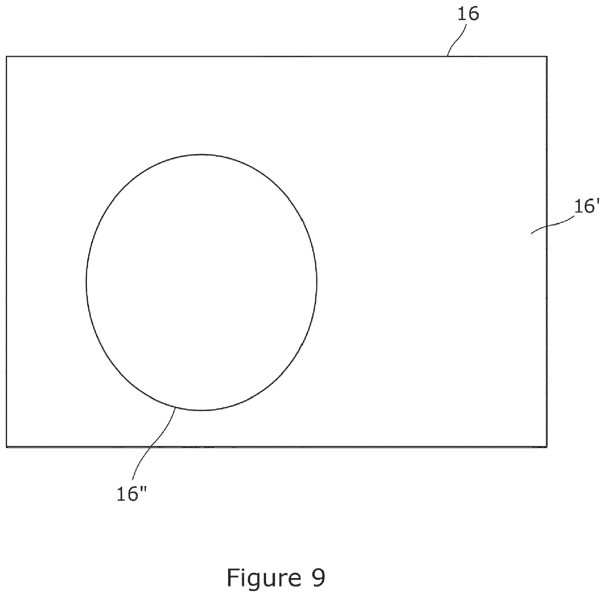

To facilitate ease of counting/detecting the functionalized beads 4 bound at the functionalized surface 9 in the second incubation event, and performing this task with increased accuracy, the ultrasonic transducer 16 may be provided in a configuration in which it comprises an illumination access feature. Such a configuration is shown in Figure 9 showing a top view of the ultrasonic transducer 16. As can be seen from Figure 9, the illumination access feature comprises at least one of: a hole 16" formed in the ultrasonic transducer 16 that may at least be partially aligned with an illumination source that is used for counting the functionalized beads 4. In addition, or alternatively, a body of the ultrasonic transducer 16 may comprise, in whole or in part, a material 16' that is transparent to radiation emitted by the illumination source. While the configuration of the ultrasonic transducer 16 shown in Figure 8 is particularly suited for use with the implementation of Figures 8A and 8B, it is not limited thereto and may be used in any embodiment of the present invention.

### Changing the geometry of the boundary surface

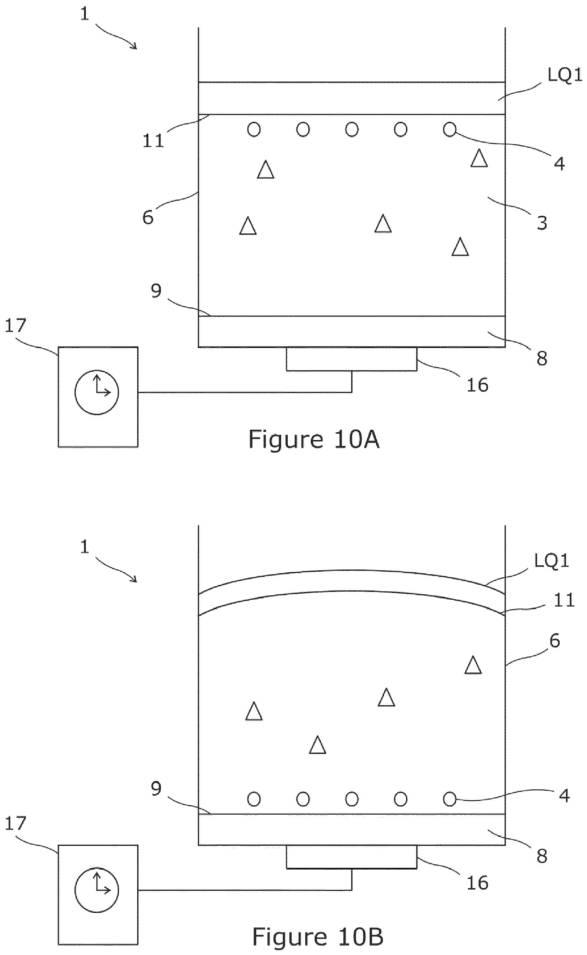

Reference is now made to Figures 10A and 10B, which show an implementation of the microfluidic device 1 where a tunable liquid lens LQ1 is provided at and encompasses the boundary surface 11. The liquid lens LQ1 comprises a host liquid 3 - oil film interface/meniscus that is tunable via an applied voltage.

By a combination of providing the liquid lens LQ1 and the operational frequency of the ultrasonic transducer 16 being set to a resonant mode, a first acoustic standing wave configuration is produced with nodes closer to the boundary surface 11. The functionalized beads 4 are trapped in these nodes in the first incubation event. This scenario is illustrated in Figure 10A.

After the predetermined time-period associated with the first incubation event is completed, tuning of the liquid lens LQ1 is performed by the voltage applied thereto. Accordingly, a curvature of the boundary surface 11 is changed, this being depicted as a concave shape in the example of Figure 10B. This results in a second acoustic standing wave configuration being produced with nodes at the functionalized surface 9, where the functionalized beads 4 are captured as can be seen from Figure 10B.

### Receptacle configuration

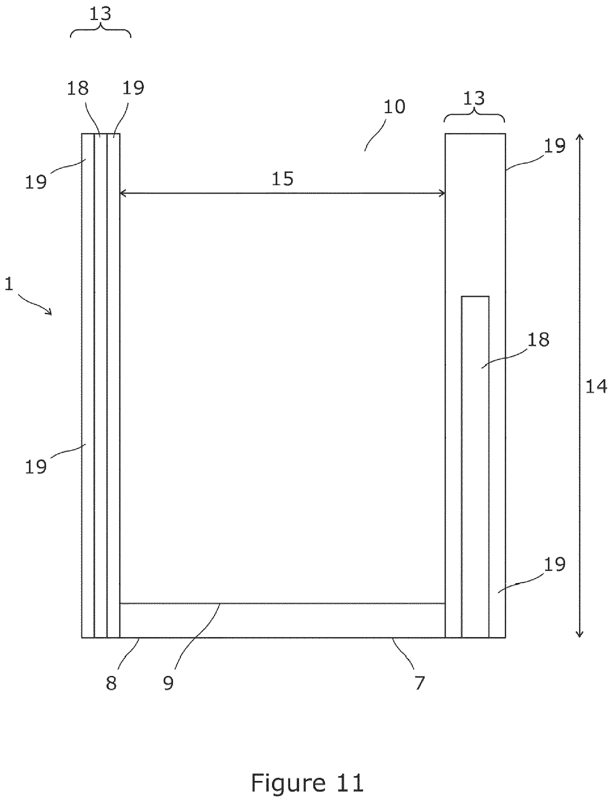

The receptacle 6 may be provided in different configurations in an embodiment of the present invention as discussed here below with reference to Figures 11, 12 and 13.

### Slots

Turning now to Figure 11, which shows how receptacle walls 13 may be configured in an embodiment of the present invention. To facilitate increased lateral confinement and/or reduced loss through the receptacle walls 13 of either of the first and second acoustic wave configurations, which are produced in relation to corresponding first and second incubation events, the receptacle walls 13 may be provided with slots 18. In this sense, the receptacle walls 13 may be provided in a multi-layered configuration comprising at least one slot 18 arranged between two layers of receptacle wall material 19, each comprising a solid/polymer material, for example. The slots 18 may be formed either fully or partially in the receptacle walls 13. The slots 18 may comprise air or indeed any other gaseous medium that at least facilitates relatively strong mode confinement of the first and second acoustic wave configurations laterally within the receptacle 6. This is because a change in acoustic impedance at an interface between the air/gas filled slot 18 and adjacent receptacle wall material 19 provides reflective boundary conditions for lateral confinement modi of the first and second acoustic wave configurations in an embodiment of the present invention. For ease of understanding, only structural aspects of the receptacle 6 that are relevant for understanding the slots 18 aspect are shown in Figure 11.

The full and partial slot configurations may be implemented individually, or in combination as shown in Figure 11. The slots 18 may be used to advantage to ensure that the first and second acoustic wave configurations have respective node patterns in x-y planes/potential energy minima such that particulates incubate without clumping together. Accordingly, the target analyte concentration in the host liquid 3 may be determined with increased accuracy.

In an embodiment of the present invention, the receptacle geometry/dimensional configurations 14, 15 combined with the acoustic impedance change that may be caused to occur across the boundary surface 11 determine the eigenmodes = acoustic standing wave configurations that are created in the first and second modes of device operation at a specific operational frequency of the ultrasonic transducer 16. Driving the ultrasonic transducer 16 at different resonant frequencies generates different eigenmodes. In addition, the boundary conditions, comprising the change in acoustic impedance across the boundary surface 11, take part in determining the eigenmodes. This is used for switching from the first to second mode of device operation by producing corresponding first and second acoustic standing wave configurations as described with reference to at least Figures 4, 6, 7 and 8. They differ in their positioning within the receptacle 6 and particularly where their nodes are positioned relative to the functionalized surface 9. In contrast, and as described earlier in relation to Figure 4 combined with Figure 5, this switch can also be performed by driving the ultrasonic transducer 16 at an off-resonant frequency. In this way completely different wave configurations are produced: a first acoustic standing wave configuration in the first mode of device operation and a second, acoustic travelling wave configuration in the second mode of device operation.

### Geometry

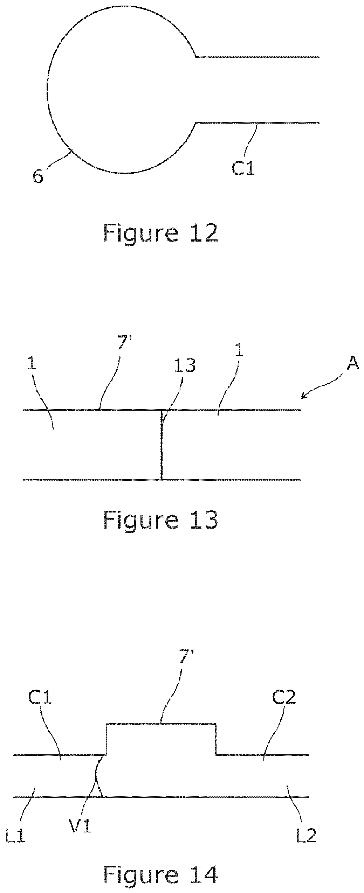

As shown in Figure 12, which shows a top view of the receptacle 6, it may be implemented in a circular form. For access, at least a conduit C1 may also be provided in this case.

The microfluidic device 1 according to an embodiment of the present invention has been shown as occurring individually thus far. However, and as explained above with reference to Figure 7 and as now illustrated by Figure 13, it may be incorporated as part of an array A of the same or other microfluidic devices 1. In the example shown in Figure 13, the different microfluidic devices 1 may be separated from each other via their respective receptacle walls 13 or dedicated separating walls therebetween.

Referring to Figure 14, and in another variation of the receptacle configuration shown in Figure 7, the receptacle 6 may be provided with more than one conduit C1, C2, at least one of which may be fitted with a vent V1. Conduits C1, C2 may respectively correspond to linking regions L1, L2 that link adjacent microfluidic devices 1 in an array format A. Correspondingly provided vents V1 may facilitate flexible and/or controllable access to fluids, for example to increase the volume of the host liquid 3 as done in Figure 7.

As can be clearly seen from Figures 13 and 14, an embodiment of the present invention can be provided such that the receptacle 6 is provided with another closed surface 7' disposed vertically opposite to the closed surface 7/base of the receptacle 6.

A corresponding method aspect is also provided according to an embodiment of which there is provided a method of determining a concentration of a target analyte 2 in a host liquid 3 using functionalized beads 4, the method comprising the steps of: providing at least a receptacle 6 that is configured to receive the host liquid 3 and the functionalized beads 4, implementing the receptacle 6 to have a closed surface 7, in relation to which a functionalized surface 9 is provided that is in communication with the host liquid 3, and to define a boundary surface 11 in respect of the host liquid 3, and positioning an ultrasonic transducer 16 relative to a region of the receptacle 6 and operating it to produce acoustic waves in the host liquid 3, the method further comprising the steps of: configuring at least an acoustic boundary condition at a distinct region of the receptacle 6 and operating the ultrasonic transducer 16 at an operational frequency depending on at least a dimensional configuration 14, 15 of the receptacle 6, which collectively facilitate operating in one of a first and second mode of operation, and correspondingly producing respective first and second acoustic wave configurations, so that the functionalized beads 4 are positioned differently in the receptacle 6 in respective first and second incubation events.

### List of reference numerals

- 1: Microfluidic device
- 2: Target analyte
- 3: Host liquid
- 4: Functionalized beads
- 4': Bead functionalizing material
- 5: Affinity bodies
- 6: Receptacle
- 7: Closed surface
- 8: Layer with functionalized surface/sensor chip
- 9: Functionalized surface
- 9': Surface functionalizing material
- 10: Open end of receptacle
- 11: Boundary surface
- 12: Medium interfacing the boundary surface
- 12': Impedance-matched material
- 13: Receptacle wall
- 14: Receptacle wall height
- 15: Receptacle diameter
- 16: Ultrasonic transducer
- 16': Material of the ultrasonic transducer body
- 16": Hole formed in the ultrasonic transducer
- 17: Timer
- 18: Slot
- 19: Receptacle wall material
- 20: Inset Fig. 2
- 21: Isopotential lines
- 22: Inset Fig. 6
- C1, C2: Conduit
- M1, M2: Meniscus of the host liquid
- F1: Filter
- L1, L2: Linking region
- V1: Vent
- A: Array

## Claims

1. A microfluidic device (1) that is configured to determine a concentration of a target analyte (2) in a host liquid (3) using functionalized beads (4), the device (1) comprising:
at least a receptacle (6) that is configured to receive the host liquid (3) and the functionalized beads (4), the receptacle (6) being implemented to have a closed surface (7), in relation to which a functionalized surface (9) is provided that is in communication with the host liquid (3), and to define a boundary surface (11) in respect of the host liquid (3), and
an ultrasonic transducer (16) that is accessible to the microfluidic device (1) when it is in use, which can be positioned relative to a region of the receptacle (6) such as to produce acoustic waves in the host liquid (3) when it is operated, wherein:
at least an acoustic boundary condition configured at a distinct region of the receptacle (6) and an operational frequency at which the ultrasonic transducer (16) is operated depending on at least a dimensional configuration (14, 15) of the receptacle (6), collectively define respective first and second modes of device operation, in which first and second acoustic wave configurations are correspondingly produced, so that the functionalized beads (4) are positioned differently in the receptacle (6) in respective first and second incubation events.

2. The microfluidic device (1) of claim 1 wherein the acoustic boundary condition comprises one of: an acoustic impedance change across the boundary surface (11) of the host liquid (3), an acoustic impedance change across the closed surface (7) of the receptacle (6), and a geometry of the boundary surface (11).

3. The microfluidic device (1) of any preceding claim wherein the ultrasonic transducer (16) is operably coupled to a timer (17) that is configured so that the ultrasonic transducer (16) is operated for a predetermined time-period in the first mode of device operation, thereby to facilitate the first incubation event occurring for the same time duration.

4. The microfluidic device (1) of any preceding claim wherein the ultrasonic transducer (16) is configured to be operated at a resonant frequency in the first mode of device operation.

5. The microfluidic device (1) of any preceding claim that is configured to provide a medium (12) to interface with the boundary surface (11) in the first mode of device operation, to which arrangement is associated an acoustic impedance mismatch across the boundary surface (11).

6. The microfluidic device (1) of any preceding claim wherein the functionalized beads (4) comprise beads whose outer surface is coated with a bead functionalizing material (4') comprising one of: at least a type of affinity body (5) that facilitates specific binding with particles of the target analyte (2) in the host liquid, and a type of a target analyte analog (2').

7. The microfluidic device (1) of any preceding claim wherein the functionalized surface (9) comprises a surface functionalizing material (9') to which at least the functionalized beads (4) may bind in the second incubation event and that is selected according to a functionalization of the functionalized beads (4').

8. The microfluidic device (1) of any preceding claim wherein at least a wall (13) of the receptacle (6) comprises at least a slot (18).

9. The microfluidic device (1) of any preceding claim wherein the receptacle (6) comprises at least a conduit (C1, C2).

10. The microfluidic device (1) of any preceding claim that is configured to be incorporated in an array (A) when it is in use.

11. The microfluidic device (1) of any preceding claim wherein the ultrasonic transducer (16) comprises an illumination access feature (16', 16") that is at least one of: a hole (16") formed in the ultrasonic transducer (16) that is at least partially aligned with an illumination source when the microfluidic device (1) is in use, and the ultrasonic transducer (16) comprising a material (16') that is transparent to radiation emitted by the illumination source.

12. The microfluidic device (1) of any preceding claim wherein the ultrasonic transducer (16) is configured to be operated at an off-resonant frequency in the second mode of device operation.

13. The microfluidic device (1) of any of claims 1 to 11 further comprising a tunable liquid lens (LQ1) that is provided in relation to the boundary surface 11 to facilitate changing a geometry thereof in the second mode of device operation.

14. The microfluidic device (1) of any of claims 1 to 11 that is configured to facilitate provision of at least an impedance-matched material (12') in contact with one of the boundary surface (11) and the closed surface (9) of the receptacle (6), in the second mode of device operation, thereby to relatively match the acoustic impedance across that surface.

15. The microfluidic device (1) of any of claims 1 to 11 wherein the receptacle (6) is configured to facilitate an increased volume of the host liquid (3) in the receptacle (6), in the second mode of device operation, so that the boundary surface (11) defined in relation to the first incubation event is immersed in the host liquid (3) and the acoustic impedance across it is matched.

16. A method of determining a concentration of a target analyte (2) in a host liquid (3) using functionalized beads (4), the method comprising the steps of:
providing at least a receptacle (6) that is configured to receive the host liquid (3) and the functionalized beads (4),
implementing the receptacle (6) to have a closed surface (7), in relation to which a functionalized surface (9) is provided that is in communication with the host liquid (3), and to define a boundary surface (11) in respect of the host liquid (3), and
positioning an ultrasonic transducer (16) relative to a region of the receptacle (6) and operating it to produce acoustic waves in the host liquid (3), the method further comprising the steps of:
configuring at least an acoustic boundary condition at a distinct region of the receptacle (6) and operating the ultrasonic transducer (16) at an operational frequency depending on at least a dimensional configuration (14, 15) of the receptacle (6), which collectively facilitate operating in one of a first and second mode of operation, and correspondingly producing respective first and second acoustic wave configurations, so that the functionalized beads (4) are positioned differently in the receptacle (6) in respective first and second incubation events.

17. The method of claim 16 further comprising selecting the acoustic boundary condition to comprise one of: an acoustic impedance change across the boundary surface (11) of the host liquid (3), an acoustic impedance change across the closed surface (7) of the receptacle (6), and a geometry of the boundary surface (11).
